(19) Europäisches Patentamt
European Patent Office
Office européen des brevets

(11) Publication number: 0 317 956 A2

# (12) EUROPEAN PATENT APPLICATION

(21) Application number: 88119418.7

(22) Date of filing: 22.11.88

(51) Int. Cl.4: C07D 487/14 , C07H 15/252 , C07D 475/08 , C07K 7/06 , C07K 9/00 , C07H 17/04 , A61K 31/40 , A61K 31/70 , A61K 31/505 , //(C07D487/14, 209:00,209:00,203:00)

(30) Priority: 23.11.87 US 124314

(43) Date of publication of application: 31.05.89 Bulletin 89/22

(84) Designated Contracting States: AT BE CH DE ES FR GB GR IT LI LU NL SE

(71) Applicant: Bristol-Myers Company
345 Park Avenue
New York New York 10154(US)

(72) Inventor: Senter, Peter D.
211 Summit Avenue East, S421
Seattle Washington(US)

(74) Representative: Kinzebach, Werner, Dr.
Patentanwälte Reitstötter, Kinzebach und Partner Sternwartstrasse 4 Postfach 86 06 49
D-8000 München 86(DE)

## (54) Anti-tumor prodrugs.

(57) There are disclosed anti-tumor prodrug compounds that are (a) disulfide benzylcarbamate derivatives of amino (primary and secondary) containing anti-tumor drugs, and (b) disulfide benzylcarbonate derivatives of hydroxyl containing anti-tumor drugs. The prodrug compounds release active, free drug as a result of undergoing reductive cleavage of the disulfide moiety and subsequent chemical fragmentation.

EP 0 317 956 A2

# ANTI-TUMOR PRODRUGS

## BACKGROUND OF THE INVENTION

### Field of the Invention

This invention relates to anti-tumor prodrugs, that is, a form of anti-tumor drugs which is capable of undergoing change in the host so as to release active, free drug. More particularly, this invention relates to (a) disulfide benzylcarbamate derivatives of amino (primary and secondary) antitumor drugs, and (b) disulfide benzylcarbonate derivatives of hydroxyl containing antitumor drugs which are capable of undergoing reductive cleavage of the disulfide moiety and subsequent chemical fragmentation so as to release active, free drug.

Reference is made to the European patent application of the same applicant (inventor Peter D. Senter) filed on the same date as the present application and entitled "Drug-Monoclonal Antibody Conjugates" (internal file number M/29 240). The disclosure of said application is incorporated herein in its entirety.

### Related Art

A considerable amount of research in the past several years has been focussed on the development of antitumor agents which have a high degree of selectivity for tumor versus normal tissue. Currently, a major limitation in the use of chemotherapeutic agents is their dose-limiting toxicity. An approach to overcome this problem has been to devise drugs that exploit the differences between neoplastic and normal cells (1).

It has been shown that solid tumors oftentimes have inadequate vascularization and may exist in oxygen-deficient, or hypoxic states (2,3). Additionally, enhanced levels of reducing agents such as NADH, NADPH and glutathione have been associated with human tumor cell lines (4-6). These observations have provided the impetus for much research towards the development and understanding of bioreductively activated anti-cancer drugs that are capable of enhanced activity under hypoxic conditions (2,3,7). Cytotoxic agents that are preferentially activated in the reducing environment of tumor cells may be of considerable therapeutic value.

## SUMMARY OF THE INVENTION

We have discovered a new drug release strategy in which mild reducing agents are capable of effecting the elimination of amino-containing drugs from benzylcarbamate disulfide drug derivatives. A series of (a) disulfide benzylcarbamate prodrugs of amino (primary and secondary) containing anti-tumor drugs and (b) disulfide benzylcarbonate derivatives of hydroxyl-containing anti-tumor drugs have been conceived, made and investigated for *in vitro* cytotoxicity on human tumor cell lines and for activity in tumor-bearing mice.

### Description of the Figures

Fig. 1 illustrates the synthesis of a model disulfide benzylcarbamate.

Fig. 2 illustrates the pathway for elimination of p-nitroaniline upon treatment of the benzylcarbamate anilide derivative with dithiothreitol.

Fig. 3 illustrates a series of mitomycin and daunomycin derivatives as prodrugs according to this invention.

In the Examples and Tables, compound numbers refer to corresponding compounds indicated in the Figures.

## DETAILED DESCRIPTION OF THE INVENTION

This invention is an anti-tumor prodrug compound having the general structural formula

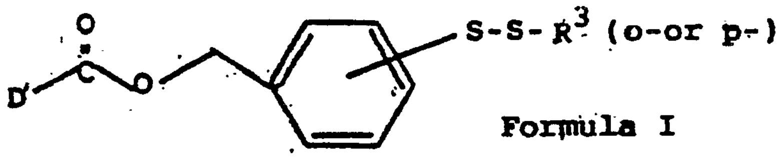

Formula I

wherein:

D is an anti-tumor drug moiety having pendant to the backbone thereof a chemically reactive functional group, by means of which the drug backbone is bonded to the disulfide benzyloxycarbonyl group, derived from the group consisting of a primary amino group represented by the formula $R^1NH$-, a secondary amino group represented by the formula $R^1R^2N$-, and a alcohol group represented by the formula $R^1O$-;

$R^1$, when $R^1$ and $R^2$ are independent, is the backbone of said drug moiety when D is derived from the group consisting of a primary amino group and a secondary amino group,

$R^2$, when $R^1$ and $R^2$ are independent, is selected from unsubstituted and substituted, and branched and straight-chain alkyl groups having 1-10 carbon atoms wherein the substituent is selected from 1 to 3 alkoxy groups having 1 to 3 carbon atoms and 1 to 3 halo groups; unsubsituted and substituted phenyl wherein the substituent is selected from 1 to 3 alkyl groups having 1 to 3 carbon atoms, 1 to 3 alkoxy groups having 1 to 3 carbon atoms, and 1 to 3 halo groups; and unsubstituted and substituted phenalkyl wherein the phenyl moiety, when substituted, is substituted as defined above in the case of substituted phenyl and the alkyl moiety is a polyalkylene group having 1 to 3 carbon atoms;

$R^1$ and $R^2$, when taken together, represent the backbone of the drug moiety, D, having a divalent group chemically bonded to the nitrogen atom constituting said secondary amino group; and

$R^3$ is an organic functional group that is compatible with the disulfide linkage and the drug moiety, D, selected from the group consisting of unsubstituted and substituted, and branched and straight-chain alkyl having 1 to 10 carbon atoms wherein, when substituted, the alkyl substituent is as defined above in the case of substituted alkyl, unsubstituted and substituted phenyl and phenalkyl wherein the phenyl moiety, when substituted, is substituted as defined above in the case of substituted phenyl and the alkyl moiety is a polyalkylene group having 1 to 3 carbon atoms, unsubstituted and substituted heteroaryl selected from the group consisting of unsubstituted and substituted pyridyl, naphthyridyl, quinolyl, isoquinolyl, and indolyl, and unsubstituted and substituted heterocyclyl selected from the group consisting of pyrrolyl, pyrrolidyl, imidazolyl, imidazolidyl, piperidyl, morpholyl, furyl, and thienyl; said substituent on said alkyl group being selected from 1 to 3 alkoxy group having 1 to 3 carbon atoms and from 1 to 3 halo groups, and said substituent on said phenyl, phenyl moiety of phenalkyl, heteroaryl and heterocyclyl groups being selected from 1 to 3 alkyl groups having 1 to 3 carbon atoms, from 1 to 3 alkoxy groups having 1 to 3 carbon atoms and from 1 to 3 halo groups; and

The substitution position of the group, -S-S-$R^3$, on the phenyl ring of the benzylcarbamate moiety is selected from the ortho- and para-positions.

Representative of said amino group-containing drugs are mitomycin-C, mitomycin-A, daunomycin, adriamycin, aminopterin, actinomycin, bleomycin, and derivatives thereof; and, representative of said alcohol group-containing drugs is etoposide.

The abbreviations used are as follows: MMC, mitomycin C; MMA, mitomycin A; DAU, daunomycin; PBS, phosphate buffered saline; T/C, median survival time of drug-treated (T) mice divided by the median survival time of tumor control mice (C) X 100; i.p., intraperitoneally; i.v. intravenously; HPLC, high pressure liquid chromatography; DDT, dithiothreitol; PNA, p-nitroaniline.

The term "prodrug" as used in this application refers to a precursor or derivative form of a pharmaceutically active substance that is less cytotoxic to tumor cells compared to the parent drug and is capable of being enzymatically activated or converted into the more active parent form [see, e.g., D.E.V. Wilman, "Prodrugs In Cancer Chemotherapy," Biochemical Society Transactions, 14, pp. 375-382 (615th Meeting, Belfact 1986) and V. J. Stella et al., "Prodrugs: A Chemical Approach To Targeted Drug Delivery," Directed Drug Delivery, R. Borchardt et al. (ed.), pp. 247-267 (Humana Press 1985).]

In another aspect this invention is a method for delivering to the site of solid tumors in a mammal having enhanced levels of endogenous reducing agents including at least one member of the group of

NADH, NADPH and glutathione, an active anti-tumor drug having pendant to the backbone thereof a chemically reactive functional group selected from the group consisting of a primary amino group represented by the formula, $R^1NH$-, a secondary amino group represented by the formula $R^1R^2N$-, and an alcohol group represented by the formula $R^1O$- wherein $R^1$ and $R^2$ are as defined above, comprising the steps of:

(a) administering to the mammal an antitumor-effective amount of an antitumor prodrug compound having formula I,

(b) contacting the antitumor prodrug from step (a) with endogenous reducing conditions, and

(c) permitting the anti-tumor prodrug to undergo reductive cleavage to release free drug from the prodrug compound.

The prodrug compounds according to this invention may be provided for use according to the method of this invention to treat a host, particularly a mammalian host such as, for example, an experimental animal host, affected by a malignant tumor, as a pharmaceutical composition. The pharmaceutical composition comprises an antitumor effective amount, i.e. a tumor growth-inhibiting amount, of the prodrug compound according to this invention, and a pharmaceutically acceptable carrier and, optionally, conventional pharmaceutically acceptable excipients and adjuvants.

The pharmaceutical carrier may be solid or liquid to provide solid or liquid compositions. Solid form compositions suitable for oral administration include powders, tablets, capsules, caplets, dispersible granules, and cachets. Suitable solid carriers include at least one carrier substance which may function only as a carrier or may in addition serve a further function such as a diluent, flavoring agent, solubilizer, lubricant, suspending agent, binder, tablet disintegrating agent, encapsulating agent and the like. Inert solid carriers include, to name but a few, magnesium carbonate and stearate, talc, sugar, lactose, pectin, dextrin, starch, gelatin, cellulosic materials, and the like. The compounds according to the invention may be provided as sterile soluble compounds or compositions, including solutions and suspensions and emulsions, thereof which can be dissolved in sterile water or other liquid medium for oral administration or for parenteral administration. Examples of liquid carriers suitable for oral administration include water, alcohol, polypropylene glycol, polyethylene glycol and mixtures of two or more of the above. Examples of liquid carriers suitable for parenteral use include water-for-injection, physiological saline, and other suitable sterile injection media. Suitable buffers for use with the liquid carrier to provide, generally, a suitable buffered isotonic solution include trisodium orthophosphate, sodium bicarbonate, sodium citrate, N-methylglucamine, L(+)-lysine, and L(+)-arginine to name but a few representative buffering agents.

The pharmaceutical composition will contain an amount of at least one compound of Formula I or mixture of one or more of said compounds or mixture thereof with another antitumor agent. The antitumor effective amount of compound of Formula I may be varied or adjusted widely depending upon the particular application, the form, the potency of the particualr compound used, and the desired concentration of compound in the composition. Generally, the amount of active component will range between about 0.5-90% by weight based on total weight of composition.

In thereapeutic use for treating a mammalian host, for example an experimental animal host, affected by a malignant tumor, the compound of this invention will be administered in an amount effective to inhibit the growth of the tumor, that is, a tumor growth-inhibiting amount of dosage. Generally, the tumor growth-inhibiting amount will be in the range of about 0.1 to about 15 mg/kg of animal body weight/day. It is to be understood that the actual preferred dosage of compound will vary widely depending upon the requirements of the animal being treated, the composition being used, and the route of administration. Many factors that modify the action of the anti-neoplastic agent will be taken into account by one skilled in the art to which this invention pertains including, for example, age, body weight and sex of the animal host; diet; time of administration; rate of excretion; condition of the host; severity of the disease; and the like. Administration may be carried out simultaneously or periodically within the maxiumum tolerated dose. Optimal administration (or application) rates for a given set of conditions may be readily ascertained by those skilled in the art using conventional dosage determination tests.

The following examples are illustrative of the scope and utility of this invention and are not to be construed as limiting the scope of the invention. Unless otherwise indicated, all parts and percentages are by weight and temperatures are in degrees Celsius. As is mentioned above, in the following Examples and Tables, compound numbers refer to corresponding compounds indicated in the Figures.

## MATERIAL AND METHODS

Cell Lines:

HSB2 (human T cell leukemia) and Namalwa (Burkitts lymphonia) were obtained from American Type Culture Collection (Gaitesburg MD). Cells were grown in RPMI 1640 medium supplemented with 10% fetal bovine serum, penicillin and streptomycin at 37° in 5% $CO_2$ humid atmosphere.

$^3$H-Thymidine Incorporation Assay.

Cytotoxicity of mitomycin and daunomycin derivatives was measured by inhibition of DNA synthesis. Serial dilutions of drugs were made in PBS and 100 µl containing $1x10^5$ cells were added to each well. Cells were incubated for 1 hr at 37°, washed twice and resuspended in 200 µl of culture medium. After incubation at 37°C for 19 hrs, 50 µl of 1 µ Ci[6-$^3$H]-thymidine (New England Nuclear, 15Ci/mmole) was added to each well and incubated for 4 hrs at 37°C. Cells were transferred to Millititer sv plates (Millipore) and precipitated with 25% cold trichloroacetic acid (TCA). The precipitates were washed 10 times with 5% cold TCA. Filters were dried, punched and counted in Econofluor liquid scentilation fluid (New England Nuclear). All counts were corrected by subtraction of background counts.

In Vivo Experiments.

The in vivo tumor tests were performed utilizing methods described in detail in a previous publication (8). The lymphatic leukemias P-388 and L-1210 were used for initial testing of mitomycin and daunomycin derivatives respectively. Selected compounds were tested against subcutaneouly implanted B16 melanoma with treatment administered intravenously. Experimental parameters are shown in the footnotes of the accompanying tables.

Chemical Methods.

Melting points were determined on a Thomas-Hoover melting point apparatus and are uncorrected. NMR spectra of all drug derivatives were obtained on a Bruker WM 360 MHZ instrument. Routine NMR spectra were obtained on IBM NR 80 MHZ instrument. IR spectra were obtained with a Nicolet 5 DX spectrometer, and uv/vis spectra were recorded with a Hewlett Packard 8450 instument. Ortho, meta and para mercaptobenzyl alcohols, 1-3, were prepared according to the procedures of Grice and Owens (9).

Preparation of the Mixed Disulfides 4 - 7.

The sulfenyl chlorides derived from 2, 2′-dithiodipyridine and bis-(3-nitrophenyl) disulfide were prepared according to the procedure of Matsueda and Aiba (10). All traces of solvent and chlorine were removed under vacuum, and the residue was suspended in $CH_2Cl_2$ to achieve a 0.1M final concentration. Solutions (0.1M, 0.9eq.) of 1-3 in $CH_2Cl_2$ were added in one batch. After 5 minutes at room temperature, the mixture was extracted with saturated $NaHCO_3$, saturated NaCl and dried ($MgSO_4$). The product was purified by flash chromatography using 30% EtOAc in pet ether as eluant.

3-Nitrophenyl Disulfide (4):

yield 41%; mp 83-84°C; $^1$H-NMR ($CDCl_3$) δ1,75 (s-1H,OH), 4.68 (s,2H,$CH_2$OH), 7.2-7.6 (q.4H,ArH).

Ortho-2-Pyridyl Disulfide (5):

yield 64%; mp 50-52°C; H-NMR ($CDCl_3$) δ4.60 (br d, 1H, OH), 4.90 (d,2H,Ar$CH_2$), 7.0-7.8 (m,7H,ArH), 8.4-8.5 (m,1H,ArH).

Meta-2-Pyridyl Disulfide (6):

yield 86% yellow oil; $^1$H-NMR ($CDCl_3$) δ1.8 (s,1H,OH), 4.65 (s,2H,$ArCH_2$), 6.9-7.8 (m,7H,ArH) 8.35-8.60 (m,1H,ArH).

Para-2-Pyridyl Disulfide (7):

yield 55% yellow oil; $^1$H-NMR ($CDCl_3$) δ1.4-1.9 (br s, 1H,OH), 4.65 (s,2H,$ArCH_2$), 7.0-7.8 (m,7H,ArH), 8.3-8.6 (m,1H,ArH).

General Procedure for the Preparation of Carbamates 12, 17-21, 24, 25. Preparation of 24.

A solution of 137 mg (0.55 mmol) of benzyl alcohol 5 and 0.044 ml of pyridine (0.55 mmol) in 1 ml of dry dioxane was added over a 3 min period to a stirred solution of 0.032 ml (0.275 mmol) of trichloromethylchloroformate in 0.5 ml of dioxane. After stirring for 15 min, a solution of MMA (96 mg, 0.275 mmol) and triethylamine (0.153 ml, 1.1 mmol) in 4 ml of dioxane was rapidly added. After 5 min, the solvents were evaporated, and a solution of the residue in $CH_2Cl_2$ was extracted with satd. $NaHCO_3$, NaCl and dried ($MgSO_4$). The product was purified by flash chromatography on a 2x20cm $SiO_2$ column by first separating non-polar material with 30% ethyl acetate in petroleum ether (300ml), and then eluting the carbamate with 5% methanol in chloroform. The product, 24, was obtained as an amorphous red solid which was dissolved in 3 ml of $CH_2Cl_2$ and added dropwise to 30 ml of pet ether. A fine red solid was obtained (125 mg, 73%): mp 148-150 °C; $^1$H-NMR ($CDCl_3$) δ 1.80 (s,3H,$CH_3$), 3.2 (s 3H, $OCH_3$), 3.3-3.8 (m,4H), 4.05 (s,3H,$OCH_3$), 4.95 (m,1H), 5.3 (ABq,2H,$ArCH_2$), 7.1-7.4 (m,4H,ArH), 7.5-7.7 (m,3H,ArH), 8.47 (d,1H,ArH); IR (KBr), λ 3400,1570 $cm^{-1}$; uv/vis ($CH_3OH$) λ max nm (log ε) 520 (3.01), 319 (4.03), 285 (3.95).

By following substantially the precedures set forth above for Example 24, except for substituting the starting thiophenol derivative and drug indicated in the Figures.

Benzyl Carbamate Disulfide 12:

yield 41% yellow solid; mp 158-160 °C; $^1$H-NMR ($CDCl_3$) δ 1.60 (s,1H,NH), 5.25 (s,2H,$CH_2OH$), 7.2-84 (m,12H,ArH); uv/vis λ max 313 nm (log ε = 4.18).

MMC Benzyl Carbamate 17:

yield 55% blue powder; mp 100-101 °C (lit 102-104 °C) (11).

MMC Benzyl Carbamate Disulfide 18:

yield 63% blue powder; mp 96-98 °C; $^1$H-NMR (pyr-$d_5$) δ 1.90 (s,3H,$CH_3$), 3.07 (s,3H,$OCH_3$), 3.4-3.55 (m,2H), 3.8-4.05 (m,3H), 4.6-5.0 (m,3H), 4.85 (s,3H), 5.35-5.70 (m,3H), 6.8-7.7 (m,7H,ArH), 8.35 (d,1H,ArH); IR (KBr) ν 3400, 1692, 1600, 1552 $cm^{-1}$; uv/vis ($CH_3OH$) λ max 365 nm (log ε = 4.32).

MMC Benzyl Carbamate Disulfide 19:

yield 98% blue powder; mp 90-92 °C; $^1$H-NMR (pyr-$d_5$) δ 1.90 (s,3H,$CH_3$), 3.05 (s,3H,$CH_3$), 3.3-3.5 (m,3H), 3.7 (m,2H), 3.9-4.0 (m,2H), 4.5-4.8 (m,3H), 4.80 (s,3H), 5.0-5.1 (m,2H), 5.50 (dd,2H,$ArCH_2$), 6.8-7.7 (m,7H,ArH), 8.3-8.4 (m,1H,ArH); IR (KBr) ν 3400, 2920, 1690, 1600, 1550 $cm^{-1}$; uv/vis ($CH_3OH$) max 357 nm (log ε = 4.31).

MMC Benzyl Carbamate Disulfide 20:.

yield 92% blue powder; mp 99° (dec); $^1$H-NMR (pyr-$d_5$) δ 1.95 (s,3H,$CH_3$), 3.15 (s,3H,$OCH_3$), 3.4-4.2 (m,6H), 4.6-5.0 (m,2H), 5.20 (s,2H,$ArCH_2$), 5.6 (dd,1H), 6.9-7.8 (m,7H,ArH), 8.35-8.5 m,1H,ArH); IR (KBr) ν 3400, 2920, 1690, 1552 $cm^{-1}$; uv/vis ($CH_3OH$) λ max 356 nm (log ε = 4.31).

MMC Benzyl Carbamate Disulfide 21:

yield 70% blue powder; mp 97-98° C; $^1$H-NMR (pyr-$d_5$) δ 1.85 (s,3H,$CH_3$), 3.03 (s,3H,)$CH_3$), 3.17 (m,2H), 3.65 (d,1H), 3.85-3.95 (m,1H), 4.55 (d,1H), 4.70 (t,1H), 5.0-5.1 (m,2H,$ArCH_2$), 5.5 (dd,1H), 7.2-7.9 (m,7H), 8.30 (m,1H,ArH); IR (KBr) ν 3400, 2920, 2690, 1600, 1560, 1350 $cm^{-1}$; uv/vis ($CH_3OH$) λ max nm (log ε) 356 (4.32), 242 (4.50).

MMA Benzyl Carbamate Disulfide 23:

yield 43% red powder; mp 78-81° C; $^1$H-NMR (pyr-$d_5$) δ 1.83 (s,3H,$CH_3$), 3.05 (s,3H,$OCH_3$), 3.35 (m,2H), 3.7 (d,1H), 3.85 (s,3H,$OCH_3$), 3.9 (q,1H,) 4.2 (d,1H), 4.6-4.9 (m,2H), 5.08 (s,2H,$ArCH_2$), 5.46 (q,1H), 7.0-7.6 (m,5H,ArH); IR (KBr) ν 3400, 2900, 1735, 1580, 1280 $cm^{-1}$.

MMA Benzyl Carbamate Disulfide 25:

yield 69% red powder; mp 73° C (dec); $^1$H-NMR (pyr-$d_5$) δ 1.83 (s,3H,$CH_3$), 3.20 (s,3H,$OCH_3$), 3.3-4.5 (m,6H), 4.0 (s,3H,$OCH_3$), 5.2 (s,2H,$ArCH_2$). 6.9-7.7 (m,7H,ArH), 8.4-8.6 (d,1H,ArH); IR (KBr) ν 3400, 1690, 1580 $cm^{-1}$; uv/vis ($CH_3OH$) λ max nm (log ε) 520 (2.90), 3.9 (3.88), 285 (3.83).

MMA Benzyl Carbamate Disulfide 26:

A solution of 11.3 µl (0.106 mmol) of p-fluorothiophenol in 1 ml of acetone was added to a solution of 65 mg (0.106 mmol) of 23 in 3 ml of acetone over a 3 min period. After 5 min, the solvent was evaporated and the product was purified by preparative TLC using 5% $CH_3OH$ in $CHCl_3$ as eluant. The desired product was collected, dissolved in 2 ml of $CH_2Cl_2$ and precipitated by dropwise addition to 10 ml of pet ether. Yield 26 mg (39%) red powder; mp 133-135° C; $^1$H-NMR ($CDCl_3$) δ 1.80 (s,3H,$CH_3$), 3.18 (s,3H,$OCH_3$), 3.30-3.70 (m,6H), 4.05 (s,3H,$0CH_3$), 4.2-4.3 (m,2H), 4.87-4.97 (q,1H), 5.1-5.25 (q,2H,$ArCH_2$), 6.95-7.05 (t,2H,ArH), 7.2-7.4 (m,5H,ArH), 7.60 (d,1H,ArH); IR (KBr) ν 3400, 1690, 1580 $cm^{-1}$; uv/vis ($CH_3OH$) λ max 338 nm.

MMA Benzyl Carbamate Disulfide 27 was prepared from 25 as described in the synthesis of 26. Yield of red powder, 39%; mp 70° C; $^1$H-NMR (pyr-$d_5$) δ 1.70 (s,3H, $CH_3$), 3.05 (s,3H,$CH_3$), 3.3-3.4 (m,2H), 3.7 (d,1H), 3.85 (s,3H,$OCH_3$), 3.80-4.0 (m,2H), 4.2 (d,1H), 4.7 (t,1H), 5.05 (s,2H,$ArCH_2$), 5.47 (m,1H), 6.8-7.0 (m,2H,ArH), 7.3-7.5 (m,2H,ArH), 7.35 (2.4H,ArH); IR (KBr) ν 3400, 1690, 1580 $cm^{-1}$; uv/vis ($CH_3OH$) λ max 518, 319 nm.

DAU Benzyl Carbamate 29:

Benzyl chloroformate (6.3 µl, 44.3 µmol) was added to a solution of 25 mg (44.3 µmol) of DAU in 1 ml $CH_2Cl_2$ containing 12.4 µmol (88.6 µmol) of triethylamine. After 2 min at room temperature, the solution was partioned between 0.1% $CH_3COOH$ ($pH_4$) and $CHCl_3$. The $CHCl_3$ layer was washed twice with the $pH_4$ buffer to remove any unreacted DAU, then with satd. NaCl, and dried ($MgSO_4$). The product was obtained as a fine red solid (24 mg, 82%) which was unstable on silica gel: mp 60° C (dec); $^1$H-NMR ($CDCl_3$) δ 1.25 (d,3H,$CHCH_3$), 1.7-1.95 (m,3H), 2.05-2.15 (m,1H), 2.25-2.5 (m,2H), 2.40 (s,3H,$COCH_3$), 3.1 (q,2H), 3.6-3.7 (d,1H), 3.8-4.0 (m,1H), 4.05 (s,3H,$OCH_3$), 4.15-4.3 (m,1H), 4.4-4.6 (m,1H), 5.03 (s,2H,$ArCH_2$), 5.05-5.50 (m,3H), 7.1-7.5 (m,6H,ArH), 7.76 (t,1H,ArH), 8.05 (d,1H,ArH), 13.28 (s,1H,OH), 13.97 (s,1H,OH); IR (KBr) ν 3400, 1720 $cm^{-1}$; uv/vis ($CH_3OH$) λ max nm (log ε) 494 (4.11), 4.78 (4.11), 289 (3.96), 252 (4.45), 234 (4.59).

DAU Benzyl Carbamate Disulfide 30 was prepared from DAU and 7 as described in the synthesis of 24. The product was purified according to the purification of 29. Yield of red powder 69%: mp 75-78° C; $^1$H-NMR ($CDCl_3$) δ 1.25 (d,3H,$CHCH_3$), 1.7-1.9 (m,2H), 2.05-2.37 (m,3H), 2.40 (s,3H,$CH_3$), 3.10 (q,2H), 3.6-3.9

(m,2H), 4.08 (s,3H,$OCH_3$), 4.2 (d,1H), 4.4 (br s, 1H), 4.98 (s,2H,$ArCH_2$),. 4.05-545 (m,3H), 7.0-7.65 (m,8H,ArH), 7.77 (t,1H,ArH), 8.0 (d,1H,ArH), 8.4-8.45 m,1H,ArH), 13.28 (s,1H,OH), 13.97 (s,1H,OH; IR (KBr) 3400, 2920, 1715 $cm^{-1}$; uv/vis ($CH_3OH$) λ max nm (log ε) 494 (3.93), 476 (3.93), 289 (4.12), 252 (4.51), 233 (4.63).

Reaction of MMC Benzyl Carbamate Disulfides 18-20 With Dithiothreitol.

To an 83/17 $CH_3OH/H_2O$ solution at 30° C containing 18, 19 or 20 (0.81 mM), tris-(hydroxymethyl) aminomethane buffer (17mM), EDTA (0.08mM) at pH 7.2 was added 100 mM dithiothreitol (final conc. 2mM). The release of MMC was measured by HPLC using a 10 cm Whatman Partasil 5 ODS-3 reverse phase (C-18) column and the following gradient system: 30% $CH_3OH$ in 0.1% acetate (pH 6) to 95% $CH_3OH$ in 6 min; continued for 8 min; flow rate 2 ml/min; monitored at 340 nm.

An anti-tumor prodrug having the alcohol-group containing drug, etoposide, whereby the etoposide is bonded to the disulfide benzyl moiety by a carbonate linkage rather than a carbamate linkage, is prepared by following substantially the foregoing "general procedure for the preparation of carbamates" except that etoposide (162 mg, 0.275 mmol) is used in place of the MMA set forth in the foregoing example to provide the product as a white solid.

## RESULTS

Model Studies.

In order to establish that benzyl carbamates with suitably positioned disulfide substituents would undergo fragmentation of the carbamate upon disulfide reduction, model studies were undertaken using p-nitroaniline as a leaving group. The synthesis of the model benzyl carbamate 12 is depicted in Figure 1. In aqueous methanol buffered anywhere between pH 6 to 8, 12 was stable for several days. However upon treatment with dithiothreitol, the disulfide bond was reduced, and p-nitroaniline was released. The presumed pathway for the elimination of p-nitroaniline is shown in figure 2.

The course of reaction was monitored by uv/vis spectroscopy, in which 14 (λ max 371 nm) was easily distinguished from the starting material 12 (λ max 313nm). HPLC served to confirm that p-nitroaniline was released. It was demonstrated that 14 was formed in a pH-dependent manner, and that release was faster at neutral or basic pH values. It was also observed that the release of 14 was slightly faster at 37° C than at room temperature.

Syntheses and Reactivities of Prodrugs.

A series of mitomycin and daunomycin derivatives were prepared by condensation of the drugs with benzylchloroformate or with the chloroformates 8 - 11 (Fig. 3). The disulfides 26 and 27 were derived from 24 and 25 by displacement of the thiopyridyl group with p-fluorothiophenol. The drug derivatives thus obtained were only slightly soluble in water, but could easily be dissolved in aqueous organic solutions.

In buffered aqueous methanol at pH 7.2, the three derivatives of mitomycin C, 18, 19 and 20, underwent rapid reduction with dithiothreitol, but subsequent elimination of mitomycin C occurred at differing rates. Under the reducing conditions, the para-disulfide (20) released mitomycin C most quickly ($t_{1/2}$10 min), and the orthodisulfide (18) was significantly slower ($t_{1/2}$72 min). As expected, the meta-isomer did not release any mitomycin C even after 18 hours. Reduction of the daunomycin disulfide, 30, resulted in a similar fragmentation reaction, and daunomycin was released. The simple benzylcarbamates, 17, 23 and 29 were stable under the reaction conditions.

In Vitro Experiments.

The drug derivatives were tested for in-vitro activity on two human lymphoid cell lines, Namalwa and HSB-2. The cells were exposed to the drugs for 1 hr. in PBS, washed, and then incubated for 19 hr.

Viability was determined by the amount of $^3$H-Thy incorporation into DNA verses an untreated control. The IC-50 values are shown in Table 1.

The results show that the most active MMC derivatives 18, 20 and 21 were those that were capable of undergoing the thiol-mediated benzyl carbamate elimination process. In fact, these compounds were significantly more active than MMC itself. The most cytotoxic MMC prodrug was the disulfide 20, which was at least 20-fold more cytotoxic than MMC. The non-cleavable MMC derivatives 17 and 19 were much less cytotoxic than MMC. A similar trend was observed for the DAU derivatives 29 and 30. The cleavable DAU prodrug 30 was much more cytotoxic than the non-cleavable derivative 29, but was somewhat less active than DAU. All of the MMA derivatives were highly cytotoxic. Under the conditions used in the in-vitro assay, the benzyl carbamate 23 was more active than MMA.

In-Vivo Experiments.

Three murine tumor models were used to evaluate the in-vivo activities of the drugs. The therapeutic effects of optimal i.p. drug doses against i.p. administered tumors are shown in Table 2. In the MMC series, the least active agent was 17, a noncleavable MMC derivative. The most active MMC derivative was the para-benzyl carbamate disulfide, 20 which was significantly more active than MMC against P388. All of the MMA derivatives were less active than the parent drug. The two daunomycin derivatives, 29 and 30 were inactive.

Some of the MMC and MMA derivatives were tested for activity against subcutaneously implanted B16 melanoma. In these experiments, the drugs were administered i.v. on days 1,5 and 9 after implantation of the tumor. The results are shown in Table 3. The cleavable MMC prodrugs, 20 and 21, were about as active as MMC. Significantly less activity was observed for benzyl carbamate MMC, 17. MMA was found to be more active than MMC against this tumor model. The cleavable prodrugs, 24 - 27, were as active as MMA, with 24 showing slightly greater activity. The non-cleavable MMA derivative, 23, was much less active than MMA.

## DISCUSSION

There has been developed a new class of prodrugs in which drug release takes place under mild reducing conditions. The fragmentation is due to the instabilities of ortho and para-substituted mercaptobenzyl carbamates. Chemically related reactions have been observed with benzisoxazolyl carbamates under basic conditions (12) and amidobenzyl carbamates after amide-bond hydrolysis (13). These reactions are all mechanistically related, and are initiated by the release of electron density from the deprotected heteroatom into the $\pi$-system. This results in the liberation of $CO_2$ and the amine component of the carbamate. The unique features of the fragmentations demonstrated here are the use of sulfur as the heteroatom, and the expulsion of a drug in chemically unmodified, active form. We have further demonstrated a relationship between heteroatom orientation and reaction rate. Drug expulsion occurs most quickly with para orientation and is slower when the disulfide is ortho with respect to the carbamate. As expected, no fragmentation is induced upon reduction of meta-benzylcarbamate disulfides. The rate differentials between ortho and para mercaptobenzyl carbamate fragmentations may be of significance in the development of optimal release rates.

The prodrugs of MMC which were capable of fragmentation (18, 20, 21) were significantly more cytotoxic than MMC itself, while the stable derivatives (17 and 19) were much less cytotoxic (Table 1). Similarly, the DAU prodrug, 30 was more active than the stable derivative 29. This indicates that the release of MMC and DAU from the prodrugs is required for maximal cytotoxic effect. The fact that 18, 20 and 21 have lower IC-50 values than MMC might suggest that they are taken up more efficiently, or release other cytotoxic agents such as thioquinone methides during fragmentation (Fig. 2).

It is noteworthy that a correlation between drug release and cytotoxicity was not observed for the MMA derivatives. The non-cleavable MMA carbamate, 23, was more cytotoxic than MMA or any of the other MMA prodrugs. MMA has been shown to differ from MMC in a number of significant ways. The drug has a much lower reduction potential and is susceptible to nucleophilic attack at the 7-methoxy position (14). It is therefore possible that the drug exerts its cytotoxic effect through pathways involving reduction or alkylation, even with substitutents on the aziridinyl nitrogen. Alternatively, it is possible that under the conditions used for the in-vitro assays, the carbamate on 23 is hydrolyzed.

The cytotoxic effects of the MMC prodrugs correlate well with the P-388 experiments (Table 2). The non-cleavable MMC derivatives, 17 and 19 were less active than MMC against P-388, consistant with what was observed in-vitro. Higher activities were found for the cleavable prodrugs, 18 20 and 21. The para-substituted MMC benzylcarbamate, 20 was not only 20-25 times more cytotoxic than MMC in tissue culture, but also significantly more active in the P-388 tumor model.

All of the cleavable MMC and MMA prodrugs tested against subcutaneously implanted B16 melanoma showed antitumor activity (Table 3). The non-cleavable derivatives, 17 and 23 had little activity. The fact that there is little difference between the cleavable prodrugs and parent drugs against B16 may indicate that the prodrugs undergo fragmentation when administered systemically. Such breakdown is apparently not as pronounced in the i.p. tumor-drug administration route, since one drug, 20, was significaltly more active than MMC (Table 2). These findings might suggest that improved activity of i.v. administered prodrugs would be obtained with more stable disulfide derivatives. The stability of disulfides in-vivo can be affected by electronic and steric factors. For example, it has been shown that steric hindrance can stabilize disulfide bonds in antibody-ricin A chain immunoconjugates (15). The in-vivo stabilities of the prodrugs reported here may be similarly controlled.

Accordingly, there has been discovered and developed a new class of antitumor drug derivatives that undergo fragmentation upon disulfide bond reduction. It has been shown that such fragmentation leads to the release of chemically unmodified parent drug. The prodrugs are at least as cytotoxic as parent drug in-vitro, and several of them have pronounced antitumor activity in-vivo. It is possible that the release strategy presented here will prove useful for the development of drug derivatives that can selectively act on cells having enhanced levels of reducing agents or on tissues in hypoxic environments.

## REFERENCES


1. Wilman, D.E.V. (1986) Biochem. Soc. Trans. 14, 375-382
2. Sartorelli, A.C. (1986) Biochem. Pharm. 35, 67-69
3. Fracasso, P.M. and Sartorelli. A.C. (1986) Cancer Research 46, 3939-3944
4. Russo, A., DeGraff, W., Friedman, N. and Mitchell, J.B. (1986) Cancer Research 46, 2845-2848
5. Degraff, W.G., Russo, A. and Mitchell, J.B. (1985) J. Biol. Chem. 260, 8312-8315
6. Lemasters, J.J., Ji, S., Thurman, R.G. (1981) Science 213, 661-663
7. Moore, H.W., Czerniak, R. and Hamdan, A. (1986) Drugs Exptl. Clin. Res. 12, 475-494
8. Bradner, W.T., Rose, W.C., Schurig, J.E., Florczyk, A.P., Huftalen, J.B., and Catino, J.J. (1985) Cancer Res. 45, 6475-6481
9. Grice, R. and Owens, L.N. (1963) J. Chem. Soc. 1963, 1947-1954
10. Matsueda, R. and Aiba, K. Chem. Lett. (1978) 951-952
11. Sasaki, H., Mukai, E., Hashida, M., Kimura, T. and Sezaki, H. (1983) Int. J. Pharmaceuktics 15, 49-59
12. Kemp, D.S. and Hoyng, C.F. (1975) Tetrahedron Lett. 1975, 4625-4628
13. Carl, P.L., Chakrovarty, P.K. and Katzenellenbogen, J.A. (1981) J. Med. Chem. 24, 479-480
14. Iyengar, B.S., Cheng, H., Remers, W.A. and Bradner, W.T. (1981) J. Med. Chem. 24, 925-981
15. Worrell, N.R., Cumber, A.J., Parnell, G.D., Mirza, A., Forrester, J.A., and Ross, W.C.J. (1986) Anti-Cancer Drug Design 1, 179-188

Table 1

| IC-50 VALUES OF ANTITUMOR AGENTS[a] | | |
|---|---|---|
| DRUG | IC-50 (molar conc.) | |
| | NAMALWA | HSB-2 |
| 16 | $5x10^{-6}$ | $4x10^{-6}$ |
| 17 | $1x10^{-5}$ | $8x10^{-6}$ |
| 18 | $2x10^{-6}$ | $2x10^{-6}$ |
| 19 | $> 10^{-5}$ [b] | $> 10^{-5}$ [c] |
| 20 | $2x10^{-7}$ | $2x10^{-7}$ |
| 21 | $7x10^{-8}$ | $1x10^{-7}$ |
| 22 | $4x10^{-9}$ | $1x10^{-8}$ |
| 23 | $7x10^{-10}$ | $2x10^{-9}$ |
| 24 | $2x10^{-8}$ | $4x10^{-8}$ |
| 25 | $9x10^{-9}$ | $2x10^{-8}$ |
| 26 | $2x10^{-8}$ | $5x10^{-8}$ |
| 27 | $1x10^{-8}$ | $4x10^{-8}$ |
| 28 | $3x10^{-7}$ | $2x10^{-7}$ |
| 29 | $2x10^{-5}$ | $3x10^{-6}$ |
| 30 | $1x10^{-6}$ | $6x10^{-7}$ |

a. Cells exposed to drug for 1h at 37° C in PBS. See Material Methods section for details
b. 32% kill at $10^{-5}$M
c. 26% kill at $10^{-5}$M

TABLE 2

| EFFECT OF MITOMYCIN AND DAUNOMYCIN DERIVATIVES ON P388 AND L1210 LEUKEMIA[a] | | |
|---|---|---|
| Drug | Optimum Dose mg/kg | Maximum Effect $T/C_b$ |
| 16 | 3.2 | 266 |
| 17 | 12.8 | 144 |
| 18 | 51.2 | 195 |
| 19 | 51.2 | 185 |
| 20 | 25.6 | 353 |
| 21 | 12.8 | 222 |
| 22 | 3.2 | 180 |
| 24 | 0.8 | 116 |
| 25 | 3.2 | 153 |
| 26 | 3.2 | 163 |
| 27 | 12.8 | 163 |
| 28 | 3.2 | 154 |
| 29 | 9.0 | 100 |
| 30 | 3.0 | 100 |

a. Tumor inoculum: $10^6$ ascites cells implanted i.p. single injection of drug, day 1. Compounds 16-27 tested against P388; 28-30 tested against L1210. Duration of experiment was 30 days.
b. T/C = median survival time (treated/control) X 100.

TABLE 3

| EFFECT OF MITOMYCIN DERIVATIVES ON B16 MELANOMA[a] | | |
|---|---|---|
| Drug | Optimum Dose mg/kg/inj | Maximum Effect T/C |
| 16 | 3.0 | 143 |
| 17 | 4.8 | 96 |
| 20 | 12.8 | 145 |
| 21 | 6.4 | 123 |
| 22 | 0.4 | 182 |
| 23 | 6.4 | 124 |
| 24 | 6.4 | 205 |
| 25 | 3.2 | 191 |
| 26 | 3.2 | 182 |
| 27 | 9.6 | 182 |

a. Tumor inoculum: Trocar piece implanted subcutaneously. Three i.v. injections, days 1, 5 and 9. Duration of experiment was 60 days.

## Claims

1. An anti-tumor prodrug compound having the general structural formula:

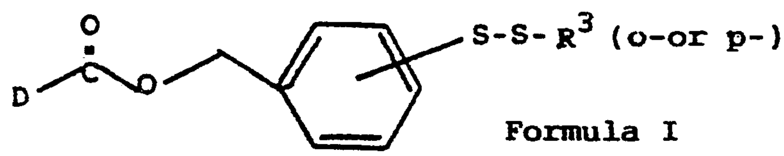

Formula I

wherein:

D is an anti-tumor drug moiety having pendant to the backbone thereof a chemically reactive functional group, by means of which the drug backbone is bonded to the disulfide benzyloxycarbonyl group, derived from the group consisting of a primary amino group represented by the formula $R^1NH$-, a secondary amino group represented by the formula $R^1R^2N$-, and a alcohol group represented by the formula $R^1O$-;

R', when $R^1$ and $R^2$ are independent, is the backbone of said drug moiety when D is derived from the group consisting of a primary amino group and a secondary amino group.

$R^2$, when $R^1$ and $R^2$ are independent, is selected from unsubstituted and substituted and branched and straight-chain alkyl groups having 1-10 carbon atoms wherein the substituent is selected from 1 to 3 alkoxy groups having 1 to 3 carbon atoms and 1 to 3 halo groups; unsubsituted and substituted phenyl wherein the substituent is selected from 1 to 3 alkyl groups having 1 to 3 carbon atoms, 1 to 3 alkoxy groups having 1 to 3 carbon atoms, and 1 to 3 halo groups; and unsubstituted and substituted phenalkyl wherein the phenyl moiety, when substitued, is substituted as defined above in the case of substituted phenyl and the alkyl moiety is a polyalkylene group having 1 to 3 carbon atoms;

$R^1$ and $R^2$, when taken together, represent the backbone of the drug moiety, D, having a divalent group chemically bonded to the nitrogen atom constituting said secondary amino group; and

is an organic functional group that is compatible with the disulfide linkage and the drug moiety, D, selected from the group consisting of unsubstituted and substituted, and branched and straight-chain alkyl having 1 to 10 carbon atoms wherein, when substituted, the alkyl substituent is as defined above in the case of substituted alkyl , unsubstituted and substituted phenyl and phenalkyl wherein the phenyl moiety, when substituted, is substituted as defined above in the case of subtituted phenyl and the alkyl moiety is a

polyalkylene group having 1 to 3 carbon atoms, unsubstituted and substituted heteroaryl selected from the group consisting of unsubstituted and substituted pyridyl, naphthyridyl, quinolyl, isoquinolyl, and indolyl, and unsubstituted and substituted heteroeyclyl selected from the group consisting of pyrrolyl, pyrrolidyl, imidazolyl, imidazolidyl, piperidyl, morpholyl, furyl, and thienyl; said substituent on said alkyl group being selected from 1 to 3 halo groups, and said substituent on said phenyl, phenyl moiety of phenalkyl, heteroaryl and heterocycle groups being selected from 1 to 3 alkyl groups having 1 to 3 carbon atoms, from 1 to 3 alkoxy groups having 1 to 3 carbon atoms and from 1 to 3 halo groups; and the orientation of the group, $-S-S-R^3$, on the phenyl ring of the benzylcarbamate moiety is selected from the ortho- and para-positions.

2. A compound according to claim 1 wherein the drug moiety, D, is a member selected from the group consisting of primary amine-containing and secondary amine-containing drugs.

3. A compound according to claim 2 wherein the drug moiety, D, is a member selected from mitomycin-C, mitomycin-A, daunomycin, adriamycin, aminopterin, actinomycin, oleomycin, and derivatives thereof.

4. A compound according to claim 1 wherein the drug moiety, D, is a carbinol group represented by the formula $R^1O$-.

5. A compound according to claim 4 wherein the drug moiety, D, is etoposide.

6. A process for preparing the prodrug compounds of anyone of claims 1 to 5 which comprises

reacting ortho- or para- mercaptobenzyl alcohol with a sulfenyl chloride of the general formula $R^3SCl$, wherein $R^3$ is as defined in claim 1, to obtain a compound of the general formula:

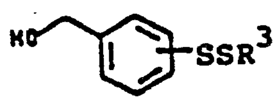

reacting the so obtained compound with trichloromethyl chloroformate in an inert organic solvent in the presence of a base, preferably pyridine, to obtain a compound of the general formula

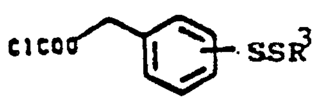

and

reacting the so obtained compound with an anti-tumor drug D, wherein D is as defined in claim 1, in an inert organic solvent and preferably in the presence of a base.

7. A pharmaceutical composition comprising at least one compound of anyone of claims 1 to 5 a process for preparing and a pharmaceutically acceptable carrier and, optionally, conventional pharmaceutically acceptable excipients and adjuvants.

8. A process for preparing the compositions of claim 7 which comprises incorporating at least one compound of claims 1 to 5 into a pharmaceutically acceptable carrier and, optionally, conventional pharmaceutically acceptable excipients and adjuvants.

9. The use of at least one compound of anyone of claims 1 to 5 for preparing a pharmaceutical composition for treating tumors.

Figure 1

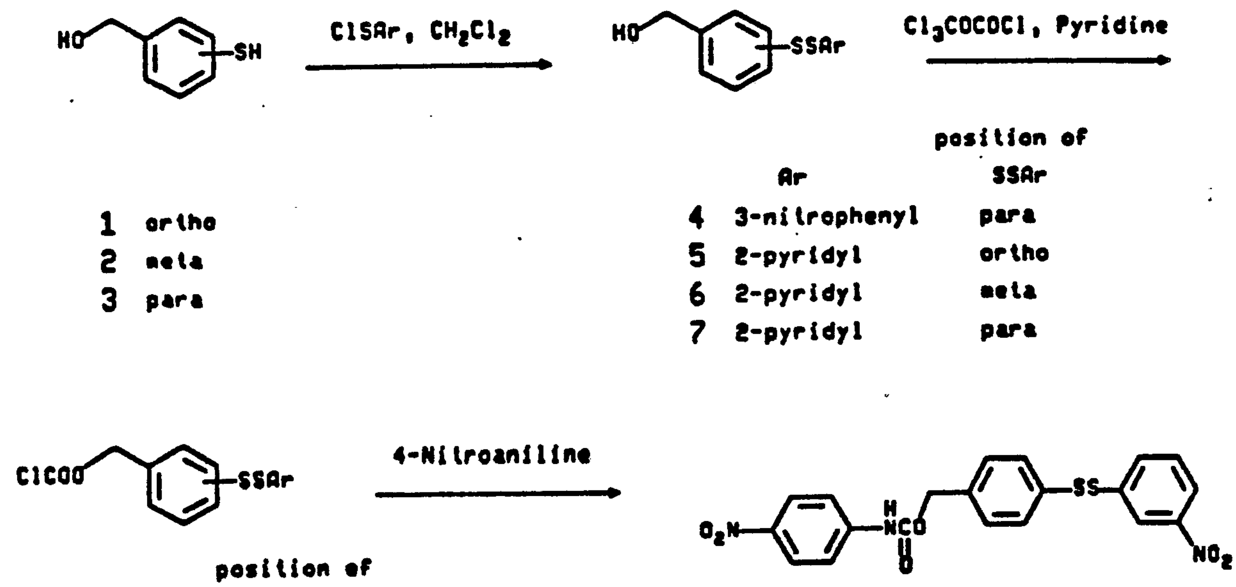


| | |
|---|---|
| 1 | ortho |
| 2 | meta |
| 3 | para |

| | Ar | position of SSAr |
|---|---|---|
| 4 | 3-nitrophenyl | para |
| 5 | 2-pyridyl | ortho |
| 6 | 2-pyridyl | meta |
| 7 | 2-pyridyl | para |

| | Ar | position of SSAr |
|---|---|---|
| 8 | 3-nitrophenyl | para |
| 9 | 2-pyridyl | ortho |
| 10 | 2-pyridyl | meta |
| 11 | 2-pyridyl | para |

12

Figure 2

12

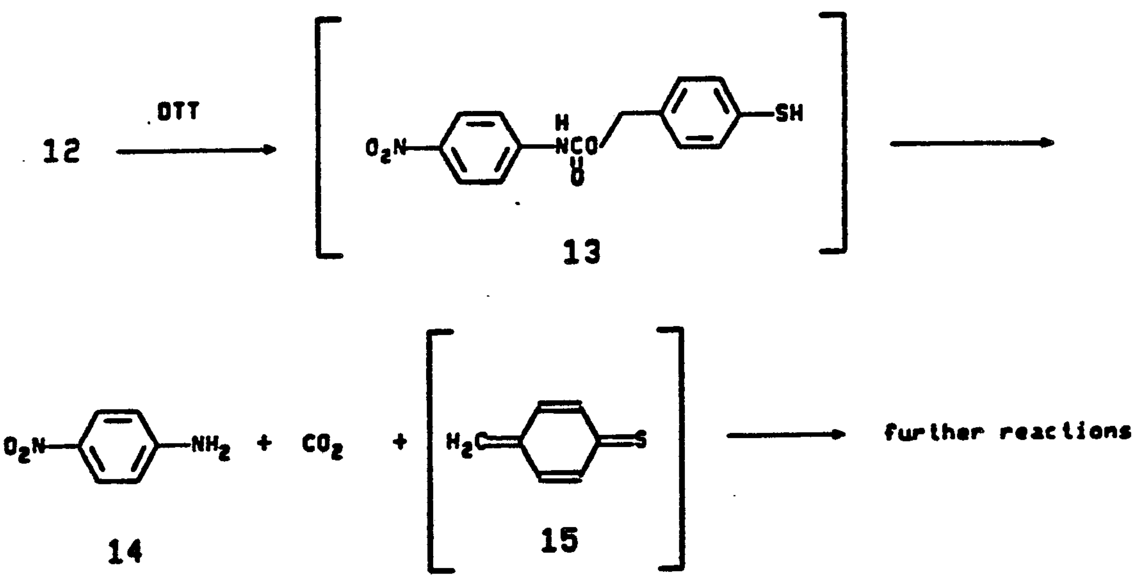


13

14

15

Figure 3

**Mitomycin C Derivatives**

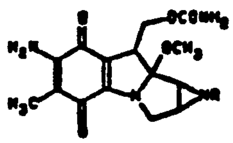

| compound | R |
|---|---|
| 16 | H (MMC) |
| 17 | 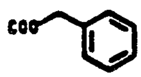 |
| 18 | 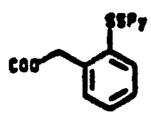 |
| 19 | 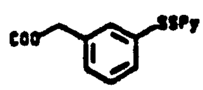 |
| 20 | 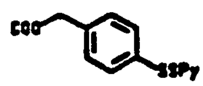 |
| 21 | 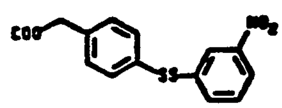 |

**Mitomycin A Derivatives**

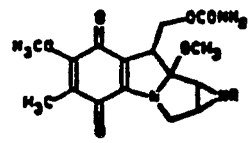

| compound | R |
|---|---|
| 22 | H (MMA) |
| 23 | 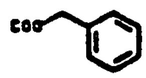 |
| 24 | 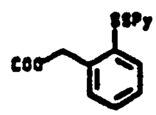 |
| 25 | 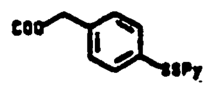 |
| 26 | 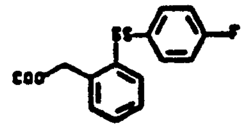 |
| 27 | 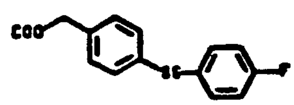 |

**Daunomycin Derivatives**

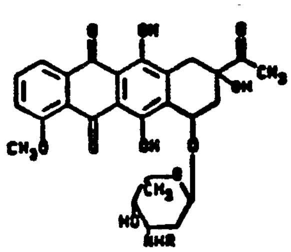

| | |
|---|---|
| 28 | H (DAU) |
| 29 | 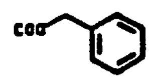 |
| 30 | 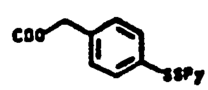 |